(19) Europäisches Patentamt
European Patent Office
Office européen des brevets

(11) EP 2 925 814 B1

(12) **EUROPEAN PATENT SPECIFICATION**

(45) Date of publication and mention of the grant of the patent:
**28.12.2016 Bulletin 2016/52**

(21) Application number: **13786607.5**

(22) Date of filing: **29.10.2013**

(51) Int Cl.:
***C08K 5/17*** *(2006.01)*

(86) International application number:
**PCT/US2013/067244**

(87) International publication number:
**WO 2014/085010 (05.06.2014 Gazette 2014/23)**

(54) **AMINOALCOHOL COMPOUNDS AS LOW VOC FREE-THAW STABILIZERS FOR PAINTS AND COATINGS**

AMINOALKOHOLVERBINDUNGEN ALS VOC-ARME EINFRIER-AUFTAU-STABILISATOREN FÜR LACKE UND BESCHICHTUNGEN

COMPOSÉS AMINOALCOOLS COMME STABILISANTS DE CONGÉLATION-DÉCONGÉLATION À FAIBLE TENEUR EN COV POUR DES PEINTURES ET DES REVÊTEMENTS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.11.2012 IN CH50062012**

(43) Date of publication of application:
**07.10.2015 Bulletin 2015/41**

(73) Proprietor: **ANGUS Chemical Company**
**Buffalo Grove, IL 60089 (US)**

(72) Inventors:
- **PEERA, Asghar A.**
  **Cary, IL 60013 (US)**
- **BHIDE, Shreyas**
  **Mumbai 400080 (IN)**

(74) Representative: **O'Neill, Michelle**
**Harrison IP**
**Westminster Business Centre**
**10 Great North Way**
**Nether Poppleton**
**York YO26 6RB (GB)**

(56) References cited:
**WO-A1-2008/081036**
**WO-A1-2010/126657**
**WO-A1-2010/126962**
**US-A1- 2006 260 505**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 925 814 B1

## Description

Cross-Reference to Related Applications

This application claims priority from Indian provisional application serial number 5006/CHE/2012, filed November 30, 2012, which is incorporated herein by reference in its entirety.

Field

This invention relates to aminoalcohol compounds and their use as freeze-thaw stabilizers.

Background

Many consumer and commercial formulations, such as paints and coatings and personal use formulations, often are subjected to widely varying temperatures, for instance during storage and transportation. Such varying temperatures may result in the formulation undergoing one or more freeze-thaw cycles. Freezing and thawing may have a detrimental effect on the formulation, unfavorably affecting its performance (e.g., in the case of paints and coatings, by increasing the viscosity), and sometimes rendering the formulations unusable. Simple glycols (e.g., ethylene glycol, propylene glycol) are sometimes included in formulations with the purpose of providing freeze-thaw (F/T) stability. However, these materials may not be desirable because they may be of high VOC and therefore generally not suitable for use in low VOC formulations.

The problem addressed by this invention is the provision of non-glycol based freeze-thaw stabilizers for consumer and commercial formulations, such as paints and coatings and personal care products, in need of such stabilization.

Statement of Invention

We have now found that aminoalcohol compounds of the formula I as described below function as highly efficient freeze thaw stabilizers. Advantageously the compounds exhibit low VOC and are therefore viable low VOC alternatives to conventional freeze thaw stabilizers, such as glycol compounds.

Accordingly, in one aspect, the invention relates to a method for providing freeze-thaw stability to a formulation in need of such stabilization, the method comprising including in the formulation a freeze-thaw stabilizer, wherein the freeze-thaw stabilizer is a compound of formula I:

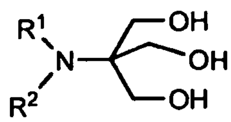

(I)

or salt thereof, wherein $R^1$ and $R^2$ are independently H, linear or branched $C_1$-$C_{10}$ alkyl, or $C_3$-$C_{12}$ cycloalkyl.

In another aspect, the invention relates to an aqueous based paint or coating comprising a freeze-thaw stabilizer, a binder, a carrier, and optionally a neutralizing agent, wherein the freeze-thaw stabilizer is a compound of formula I:

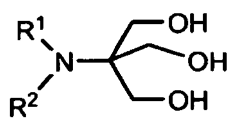

(I)

or salt thereof, wherein $R^1$ and $R^2$ are independently H, linear or branched $C_1$-$C_{10}$ alkyl, or $C_3$-$C_{12}$ cycloalkyl, and wherein the aqueous based paint or coating is glycol-free.

Detailed Description

Unless otherwise indicated, numeric ranges, for instance as in "from 2 to 10," are inclusive of the numbers defining the range (e.g., 2 and 10).

Unless otherwise indicated, ratios, percentages, parts, and the like are by weight.

"Glycol-free" and like terms mean that a formulation comprises less than 1 wt%, preferably less than 0.5 wt% and more preferably less than 0.2 wt%, of a glycol (e.g., ethylene glycol, propylene glycol) based on the weight of the formulation. The formulation can comprise some minimal amount, e.g., less than 1 wt%, of glycol for purposes other than as a freeze-thaw agent, but this amount is insufficient to impart to the formulation sufficient freeze-thaw properties so as to pass the 5-cycle freeze-thaw (F/T) test described in the examples.

"Freeze-thaw stability" means that the formulation is able to pass the 5-cycle freeze-thaw F/T test described in the examples.

"Low-VOC formulation" and like terms mean an organic volatile content of 50 grams or less per liter of formulation, such as paint or coating (water excluded) for the entire formulation. "Zero-VOC formulation" and like terms mean an organic volatile content of 5 grams or less per liter of formulation (water excluded) for the entire formulation. Total organic volatile content may be calculated by using information for the individual raw materials. Organic volatile content of raw materials (other than the aminoalcohol compounds of the invention) may be measured using well known tests, such as the EPA24 test method.

"Zero or low VOC compound" and like terms mean that a particular compound has a boiling point at atmospheric pressure of at least 280°C.

The invention provides aminoalcohols that are useful as freeze-thaw stabilizers. The aminoalcohols are compounds of the formula I:

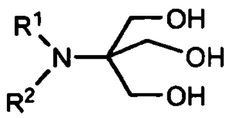

**(I)**

or salt thereof, wherein $R^1$ and $R^2$ are independently H, linear or branched $C_1$-$C_{10}$ alkyl, or $C_3$-$C_{12}$ cycloalkyl.

In some embodiments, $R^1$ in formula I is linear or branched $C_1$-$C_6$ alkyl, alternatively linear or branched $C_1$-$C_4$ alkyl, or alternatively it is methyl, ethyl, or propyl.

In some embodiments, $R^2$ in formula I is linear or branched $C_1$-$C_6$ alkyl, alternatively linear or branched $C_1$-$C_4$ alkyl, or alternatively, it is methyl, ethyl, or propyl.

In some embodiments, $R^1$ and $R^2$ are the same and are both H, or both linear or branched $C_1$-$C_6$ alkyl, alternatively both linear or branched $C_1$-$C_4$ alkyl, or alternatively both methyl, ethyl, or propyl.

Preferred compounds of formula I include: 2-amino-2-(hydroxymethyl)propane-1,3-diol, 2-(dimethylamino)-2-(hydroxymethyl)-1,3-propanediol; 2-(diethylamino)-2- (hydroxymethyl)-1,3-propanediol; 2-(dipropylamino)-2-(hydroxymethyl)-1,3-propanediol; and 2-[bis(2-methylpropyl)amino]-2-(hydroxymethyl)-1,3-propanediol. A particularly preferred compound is 2-(dimethylamino)-2-(hydroxymethyl)-1,3-propanediol.

Particularly preferred compounds of formula I include 2-amino-2-(hydroxymethyl)propane-1,3-diol (TA) and 2-(dimethylamino)-2-(hydroxymethyl)-1,3-propanediol (DMTA).

Compounds of formula I may be prepared by those skilled in the art using well known techniques or they may be purchased from commercial sources. The compounds may be used in the invention in neutral form or as acid salts. Suitable acids for the salts include, but are not limited to, hydrochloric acid, boric acid, lactic acid, pelargonic acid, nonanoic acid, neodecanoic acid, sebacic acid, azelaic acid, citric acid, benzoic acid, undecylenic acid, lauric acid, myristic acid, stearic acid, oleic acid, tall oil fatty acid, ethylenediaminetetraacetic acid and like materials.

The compounds of formula I may be used as freeze-thaw stabilizers in consumer and commercial formulations that need such stabilization. The amount of the formula I compound that should be used in a formulation for providing freeze-thaw stability can be readily determined by a person of ordinary skill in the art. By way of non-limiting example, the amount may be from 0.1 to 10 percent by weight based on the total weight of the formulation.

Preferred formulations in which the compounds of formula I may be used as freeze-thaw stabilizers include personal care products and aqueous based paints or coatings. Aqueous based paints or coatings are particularly preferred.

Aqueous based paint or coating formulations are used to provide protective and/or decorative barriers for residential and industrial surfaces, such as for floors, automobiles, exteriors and interiors of houses, and other buildings. Typically, such paint or coating formulation, in addition to comprising a freeze-thaw stabilizer, also comprises a binder and a carrier. A pigment may also be included where a pigmented paint or coating is desired. The formulation may contain other additives commonly used in paints and coatings including, but not limited to, leveling agents and surfactants, thickeners, rheology modifiers, co-solvents, corrosion inhibitors, defoamers, co-dispersants, and biocides.

Binders are included in the paint and coating formulations to provide a network in which the other additives are

dispersed and suspended. Binders also function as the primary film forming component of the finished coating, provide integrity and adhesion for the coated film and overall protect the substrate from the external environment. Generally, there are two classes of binders: latex binders are used in aqueous based formulations, and alkyd-based binders are used in non-aqueous formulations, ultimately resulting in latex paints and coatings and alkyd paints and coatings, respectively.

In latex based paint and coating formulations, the binders are typically prepared by free radical initiated aqueous emulsion polymerization of a monomer mixture containing alkyl acrylate (methyl acrylate, ethyl acrylate, butyl acrylate and/or 2-ethylhexylacrylate), alkyl methacrylate, vinyl alcohol/acetate, styrene, and/or acrylonitrile and ethylene type monomers. The amount of the binder in the formulations of the invention can be the amount conventionally used in paint and coating formulations. By way of non-limiting examples, the amount of binder solids may be from about 2% to about 75%, alternatively from about 5% to about 65%, or alternatively from about 20% to about 55%, by weight based on the total weight of the formulation.

The formulations also contain a carrier in which the formulation ingredients are dissolved, dispersed, and/or suspended. In the aqueous based formulations of the preferred embodiment of the invention, the carrier is usually water, although other water-based solutions such as water-alcohol mixtures and the like may be used. The aqueous carrier generally makes up the balance of the formulation, after all the other ingredients have been accounted for.

Neutralizers may be included in aqueous based paints or coatings in order to neutralize residual acid moieties or to raise the pH to a desired value, sometimes between about 8 and 10. Suitable neutralizers are well known in the industry and include, without limitation, ammonia, amino-2-methyl-1-propanol (AMP), monoethanolamine (MEA), inorganic bases such as potassium hydroxide and sodium hydroxide. Compounds of formula I may also be used as neutralizers, in which they may provide the dual function of freeze-thaw additive and neutralizing agent.

Pigments may be included to provide hiding power and a desired color to the final coated material and may also be used to provide bulk to the paint or coating. While multiple pigments may be present in end-use paints or coatings, sometimes only white pigment, such as titanium oxide, perhaps in combination with extender pigments such as calcium carbonate and/or kaolin clay, is added in the early stages of the formation of the formulation. Any other desired pigments of various colors (including more white pigment) can optionally be added at the later stages of, or after, the formulation is completed.

Pigments may be organic or inorganic. Examples of pigments can include, but are not limited to, titanium dioxide, kaolin clay, calcined kaolin clay, carbon black, iron oxide black, iron oxide yellow, iron oxide red, iron oxide brown, organic red pigments, including quinacridone red and metallized and non-metallized azo reds (e.g., lithols, lithol rubine, toluidine red, naphthol red), phthalocyanine blue, phthalocyanine green, mono- or di-arylide yellow, benzimidazolone yellow, heterocyclic yellow, quinacridone magenta, quinacridone violet, and the like, and any combination thereof.

Paint and coating formulations may be manufactured by conventional paint manufacturing techniques, which are well known to those skilled in the art. Typically, the formulations are manufactured by a two-step process. First, a dispersion phase, commonly referred to as the grind phase, is prepared by mixing the dry pigments (if present) with other grind phase components, including most other solid powder formulation materials, under constant high shear agitation to provide a high viscosity and high solids mixture. This part of the process is designed to effectively wet and dis-agglomerate the dry materials and stabilize them in an aqueous dispersion.

The second step of the paint manufacturing process is commonly referred to as the letdown or thindown phase, because the viscous grind is diluted with the remaining formulation components, which are generally less viscous than the grind mix. Typically, the binders, any predispersed pigments, and any other paint materials that only require mixing and perhaps moderate shear, are incorporated during the letdown phase. The letdown phase may be done either by sequentially adding the letdown components into a vessel containing the grind mix, or by adding the grind mix into a vessel containing a premix of the latex resins and other letdown components, followed by sequential addition of the final letdown components. In either case, constant agitation is needed, although application of high shear is not required.

The compounds of formula I are preferably added late in the formulation (e.g., during the letdown phase of the manufacturing process, as described above).

The compounds of the invention function as zero or low VOC freeze-thaw (F/T) stabilizers. Because of their ability to function as freeze thaw stabilizers, known F/T stabilizers, such as glycols, may be eliminated from formulations, such as paints and coating. This has the advantage of potentially further reducing the VOC of a formulation.

Thus, in some embodiments the invention provides a glycol-free, low VOC formulation comprising a compound of formula I as an F/T stabilizer. Preferably, the formulation is a paint or coating. In some embodiments, a glycol-free, low VOC paint or coating formulation of the invention may comprise in weight percent (wt%) based on the weight of the formulation: 5 to 35% of a binder; 10 to 30% pigment; 0.2 to 10% compound of formula I; and 30 to 60% carrier. In some embodiments, the pH of the formulation is in a range between 7 and 13. In some embodiments, 2-amino-2-(hydroxymethyl)propane-1,3-diol (TA) or 2-(dimethylamino)-2-(hydroxymethyl)-1,3-propanediol (DMTA) are preferred F/T stabilizers.

In some embodiments, it is desirable for the paint or coating to contain 2-amino-2-(hydroxymethyl)propane-1,3-diol (TA) as the freeze-thaw stabilizer and, in addition to conventional components such as carriers and binders, 2-amino-2-methyl-1-propanol (AMP) as a neutralizer. In other embodiments, it is desirable for the paint or coating to contain 2-(dimethylamino)-2-(hydroxymethyl)-1,3-propanediol (DMTA) as the freeze-thaw stabilizer and, in addition to conventional components such as carriers and binders, 2-amino-2-methyl-1-propanol (AMP) as a neutralizer. It has been found that the combination of 2-amino-2-(hydroxymethyl)propane-1,3-diol (TA) or 2-(dimethylamino)-2-(hydroxymethyl)-1,3-propanediol (DMTA) together with AMP provides a synergistic effect with respect to F/T performance, allowing for lower amounts of the TA or DMTA to be used. According to this embodiment, it may be preferred for the weight ratio of 2-amino-2-(hydroxymethyl)propane-1,3-diol to AMP or 2-(dimethylamino)-2-(hydroxymethyl)-1,3-propanediol (DMTA) to AMP to range from 1:1 to 5:1.

Some embodiments of the invention will now be described in detail in the following Examples.

EXAMPLES

In the following examples, two inventive compounds, 2-amino-2-(hydroxymethyl)propane-1,3-diol (TA) and 2-(dimethylamino)-2-(hydroxymethyl)-1,3-propanediol (DMTA) are test as free-thaw stabilizers in an aqueous paint formulation and compared to compared to non-inventive systems. Tris Amino (TA) is commercially available from Angus Chemical Company. DMTA may be prepared from tris (hydroxymethyl) aminomethane and formaldehyde via a variation of the reductive methylation method described in US Patent 5,105,013. A Raney nickel catalyst is used, and methanol is the solvent. The yield of DMTA is > 98%, with a GC area % purity of > 98%. IR, NMR, and GC/MS analyses confirm the product identity.

The paint formulation used for testing is shown in Table 1.

Table 1.

| **Ingredient** | **Function** | **% by wt** | **gm** |
|---|---|---|---|
| Water | Water | 17 | 595 |
| Kathon LXE | Biocide | 0.1 | 3.5 |
| ROZONE 2000 | Biocide | 0.3 | 10.5 |
| Tego Foamex | defoamer | 0.02 | 0.7 |
| HEC | thickener | 0.5 | 17.5 |
| Orotan 731 A | Ionic dispersant | 0.6 | 21 |
| Neutralizer | Neutralizer | 0.1 | 3.5 |
| Tergitol 15S40 | Non-ionic surfactant | 0.2 | 7 |
| TiO2 R706 | pigment | 18.00 | 630 |
| Calcite MF | extender | 4.00 | 140 |
| Omyacarb | extender | 3 | 105 |
| ACRYSOL RM 5000 | Rheology modifier | 1 | 35 |
| Neutralizer | Neutralizer | 0.1 | 3.5 |
| Water | Water | 16.16 | 565.6 |
| Tego Foamex | defoamer | 0.02 | 0.7 |
| Water | Water | 1.9 | 66.5 |
| ROPAQUE ULTRA E | Opaque polymer, dry hiding | 7.00 | 245 |
| Emulsion SF 018 | Acrylic binder | 30.00 | 1050 |
| **Total** | | **100** | **3500** |

AMP (available as AMP-95® from Angus Chemical Company) is used as a neutralizer in most of the studies. The AMP is added in sequence at two different places as indicated in table 1. However, when 10% KOH is used as a neutralizer instead, only 0.1 wt% is used and it is added at once. In the examples below, "blank" refers to a paint sample that contains no post-additives and the paint sample is not subjected to any F/T cycles.

In Example 1 below, TA and DMTA is added to the paint samples as F/T post-add at 0.2 wt%, 0.3 wt%, 0.5

wt%, 1 wt% and 2 wt%. The paints in this example is neutralized with AMP-95.

In Example 2, TA and DMTA is added to the paint samples as F/T post-add at 0.2 wt%, 0.3 wt%, 0.5 wt%, 1 wt% and 2 wt%. The paints in this example is neutralized with DMTA.

In Example 3, TA and DMTA is added to the paint samples as F/T post-add at 0.2 wt%, 0.3 wt%, 0.5 wt%, 1 wt%, and 2 wt%. The paints in this example is neutralized with KOH solution.

For comparison purposes a paint sample is prepared with propylene glycol (PG) post-added at 2 wt%.

**Procedure for Freeze-Thaw Cycles.** The paint samples are kept in a freezer at -18°C for 17 h followed by a thaw at 25°C for 7 h. After the thaw, the paint in the containers is stirred and then the viscosity is measured using a Stormer viscometer. This completes 1 freeze-thaw cycle. After 4 freeze-thaw cycles the paint samples are kept on the shelf for 72 hours and afterwards the paint samples are subjected to 1 more freeze-thaw cycle. The paint samples, thus, are subjected to 5 freeze-thaw cycles.

It should be noted that, for all of the paints listed in this document, after each F/T cycle the paint samples are checked for paint flow and bit formation. It was checked that upon stirring the paint can be reformed and there is no observable phase separation. Only after these checks are successful is a paint sample considered to have "passed" the F/T cycle. Thus, draw-downs can be performed easily with the paint samples that have passed 5 F/T cycles.

Properties such as opacity, whiteness and gloss are measured on paint samples that have survived 5 F/T cycles. The paint properties are measured on paint draw-downs that are 150 μm in thickness. The Opacity and whiteness are measured using a device from Sheen Micro match Plus. The gloss is measured using a Rhopoint instrument. The color difference between the reference and sample paints is calculated in terms of

$$\Delta E = \sqrt{(\Delta L)^2 + (\Delta a)^2 + (\Delta b)^2}$$

Here, ΔL, Δa and Δb are the differences between the sample (the paint with a post-additive) and the reference (blank paint) of the CIE (International Commission on Illumination) color space L, a, b values. ΔE = 0.5 is considered to be the just noticeable difference value between the colors of the reference and the sample paints.

Example 1:

The data in example 1 outlines the viscosity change of paints (Table 2), opacity (Table 3), gloss (Table 4) and whiteness (Table 5) of paints that are neutralized with AMP and contain either TA or DMTA as F/T additive. The TA and DMTA are post added at 0.2, 0.3, 0.5, 1 and 2 wt%. The paint samples containing TA or DMTA as freeze thaw additives do not have any glycol added to them. These samples are compared with paints that contain 2 wt % of propylene glycol as a benchmark. The "blank" paint that does not contain any F/T additive such as PG, TA or DMTA does not survive more than one (1) F/T cycle and solidifies rendering it ineffectual as paint.

Table 2: Viscosity of different paint samples containing DMTA and TA after each F/T cycle and when 0.2 wt% of AMP-95 is used as neutralizer.

| Sample Number | Post-add compound | Post-additive wt% in the sample | Initial Viscosity | Viscosity after Freeze-Thaw cycles | | | | |
|---|---|---|---|---|---|---|---|---|
| | | | | 1st cycle | 2nd cycle | 3rd cycle | 4th cycle | 5th cycle |
| 1 | TA | 0.2 | 92.6 | 102 | 106.5 | 107.6 | 109.5 | 111.1 |
| 2 | TA | 0.3 | 91.9 | 103.9 | 106.4 | 108.2 | 107.1 | 105.3 |
| 3 | TA | 0.5 | 88.1 | 103.1 | 102.6 | 106 | 103.2 | 102.9 |
| 4 | TA | 1.0 | 92.6 | 111.4 | 114.5 | 114.7 | 123.1 | 116.1 |
| 5 | TA | 2.0 | 91.3 | 104.7 | 105.4 | 106.3 | 111.3 | 105.4 |
| 6 | DMTA | 0.2 | 89.1 | 102.6 | 105.6 | 108 | 109.8 | 108.9 |
| 7 | DMTA | 0.3 | 91.1 | 101.7 | 103.5 | 105.5 | 106.2 | 105.5 |
| 8 | DMTA | 0.5 | 92.2 | 103.8 | 104.8 | 106.2 | 106.8 | 105 |
| 90 | DMTA | 1.0 | 90.9 | 100.1 | 100.6 | 101 | 101.3 | 101.6 |
| 10 | DMTA | 2.0 | 90.8 | 96.6 | 96.4 | 96 | 96.9 | 96.9 |

(continued)

| Sample Number | Post-add compound | Post-additive wt% in the sample | Initial Viscosity | Viscosity after Freeze-Thaw cycles | | | | |
|---|---|---|---|---|---|---|---|---|
| | | | | 1st cycle | 2nd cycle | 3rd cycle | 4th cycle | 5th cycle |
| 11 | Propylene Glycol | 2.0 | 89 | 107.5 | 110.3 | 116.4 | 124 | 119.3 |

Post F/T viscosity of the paint samples that contain TA or DMTA post-additive at 0.3 or 0.5 wt% is at par with the viscosity of the paint samples that contain the same post-additive at 1 or 2 wt%. It can be noted from Table 2 that the post F/T viscosities of the paint samples that contain TA or DMTA post-additives as low as 0.2 wt% are lower than that of the paint sample containing PG post-additive at 2 wt%. Thus showing the effectiveness of the TA and DMTA as F/T additive.

Tables 3, 4 and 5, outline some of the basic paint properties of the paints that have undergone five (5) freeze thaw cycles. The reference paint in these measurements is a blank paint, which neither has a freeze thaw additive nor does it get subjected to any freeze-thaw cycle. There is no significant deterioration of opacity or whiteness in samples where TA or DMTA is post-added in quantities less than 1 wt%. The paint samples are considered to have retained the opacity, when the difference is within 1%. As listed in Table 3, all paint samples retain opacity post F/T. Similarly, whiteness as listed in Table 5, except for the case when TA is present at 0.2 wt%, is retained even after undergoing 5 F/T cycles. The gloss is retained post-F/T only when TA or DMTA is post-added in quantities at 0.5 wt% or greater in the paint samples.

Table 3: Opacity data on paint samples with DMTA and TA additives that are subjected to 5 F/T cycles and when 0.2 wt% of AMP-95 is used as neutralizer.

| Paint Sample with the additive, wt% | Opacity, % (Reference) | Opacity, % (Sample) |
|---|---|---|
| TA, 0.2 wt% | 95.31 | 95.98 |
| TA, 0.3 wt% | 96.23 | 96.59 |
| TA, 0.5 wt% | 96.99 | 96.62 |
| TA, 1.0 wt% | 94.99 | 95.8 |
| TA, 2.0 wt% | 95.44 | 96.21 |
| DMTA, 0.2 wt% | 96.05 | 95.76 |
| DMTA, 0.3 wt% | 95.34 | 94.8 |
| DMTA, 0.5 wt% | 96.11 | 95.73 |
| DMTA, 1.0 wt% | 96.21 | 95.21 |
| DMTA, 2.0 wt% | 96.39 | 94.44 |
| Propylene Glycol, 2 wt% | 96.33 | 95.75 |

Table 4: Gloss data on paint samples with DMTA and TA additives that are subjected to 5 F/T cycles and when 0.2 wt% of AMP-95 is used as neutralizer.

| Sample with the additive, wt% | Gloss 20°, 65°, 85° (reference) | Gloss 20°, 65°, 85° (sample) |
|---|---|---|
| TA, 0.2 wt% | 2.7, 14.9, 29.0 | 2.1, 8.9, 20.6 |
| TA, 0.3 wt% | 2.6, 15.1, 32.0 | 2.4, 11.9, 24.3 |
| TA, 0.5 wt% | 2.7, 15.4, 30.7 | 2.9, 16.5, 32.0 |
| TA, 1.0 wt% | 2.3, 12.5,28.1 | 2.4, 12.2, 26.2 |
| TA, 2.0 wt% | 2.4, 12.8,31.1 | 2.1, 17.7, 39.2 |
| DMTA, 0.2 wt% | 2.7, 13.7, 30.0 | 2.1, 8.0, 19.1 |
| DMTA, 0.3 wt% | 2.7, 15.5, 28.5 | 2.3, 10.8, 19.4 |

(continued)

| Sample with the additive, wt% | Gloss 20°, 65°, 85° (reference) | Gloss 20°, 65°, 85° (sample) |
|---|---|---|
| DMTA, 0.5 wt% | 2.7, 15.7, 30.9 | 2.6, 14.7, 25.5 |
| DMTA, 1.0 wt% | 2.6, 15.6, 31.6 | 3.1, 18.0, 32.5 |
| DMTA , 2.0 wt% | 2.6, 15.4, 28.3 | 3.6, 21.4, 34.2 |
| Propylene Glycol, 2 wt% | 2.4, 13.1, 30.1 | 2.2, 10.7, 28.1 |

Table 5: Whiteness data on paint samples with DMTA and TA additives that are subjected to 5 F/T cycles and when 0.2 wt% of AMP-95 is used as neutralizer.

| Paint Sample with the F/T additive, wt% | L (ref) | L (sample) | a (ref) | a (sample) | b (ref) | b (sample) | ΔE |
|---|---|---|---|---|---|---|---|
| TA, 0.2 wt% | 94.85 | 95.57 | -0.89 | -0.81 | 1.32 | 1.29 | 0.73 |
| TA, 0.3 wt% | 94.81 | 95.03 | -0.83 | -0.81 | 1.3 | 1.29 | 0.22 |
| TA, 0.5 wt% | 94.57 | 94.84 | -0.82 | -0.8 | 1.19 | 1.22 | 0.27 |
| TA, 1.0 wt% | 94.78 | 94.98 | -0.98 | -0.86 | 1.04 | 1.06 | 0.23 |
| TA, 2.0 wt% | 94.91 | 94.86 | -1.04 | -0.92 | 1.02 | 1.15 | 0.18 |
| DMTA, 0.2 wt% | 95.31 | 95.33 | -0.81 | -0.76 | 1.24 | 1.26 | 0.06 |
| DMTA, 0.3 wt% | 95.21 | 95.42 | -0.8 | -0.78 | 1.39 | 1.4 | 0.21 |
| DMTA, 0.5 wt% | 95.4 | 95.25 | -0.84 | -0.81 | 1.33 | 1.31 | 0.15 |
| DMTA, 1.0 wt% | 95.09 | 95.04 | -0.81 | -0.77 | 1.3 | 1.3 | 0.06 |
| DMTA, 2.0 wt% | 95.00 | 94.82 | -0.87 | -0.75 | 1.31 | 1.25 | 0.22 |
| PG, 2wt% | 95.68 | 95.65 | -1.18 | -1.62 | 1.37 | 1.24 | 0.46 |

Example 2

This example demonstrates the use of DMTA both as a neutralizer and as F/T additive in the same formulation. The use of DMTA as neutralizer has been reported previously (US 8293002 B2). However, there are no reports that have shown the multiple use of DMTA effectively acting both as a neutralizer and as F/T additive in the same system. The advantage of such a system is the ability to eliminate the use of multiple additives in the paint formulations thus simplifying the formulation and potentially reducing costs.

Table 6, outlines the viscosity changes observed in paint samples at each F/T cycle. These samples do not have any glycol added to them. The amino alcohols DMTA and TA act as F/T additives. Based on the data shown, the viscosity build-up when DMTA and TA is used as F/T additive under 1.0 wt% is significantly higher (for example, the paint sample that contains DMTA as a post-add at 0.5 wt%) and in most cases the paints do not survive all the F/T cycles. However, when DMTA and TA are added as F/T additive in quantities at or greater than 1 wt%, the paint samples survived all the five cycles. Samples that survived the F/T cycles are subjected to more performance testing. The data is summarized in Tables 6-9. Data for the samples is relative to a reference paint, which neither has a freeze thaw additive nor does it get subjected to any freeze-thaw cycle.

Table 6: Viscosity of different paint samples containing DMTA and TA after each F/T cycle and when 0.2 wt% of DMTA is used as neutralizer.

| Sample Number | Post-add compound | Post-additive wt% in the sample | Initial Viscosity | Viscosity after Freeze-Thaw cycles | | | | |
|---|---|---|---|---|---|---|---|---|
| | | | | 1st cycle | 2nd cycle | 3rd cycle | 4th cycle | 5th cycle |
| 1 | TA | 0.2 | 92.9 | gel | gel | gel | gel | gel |
| 2 | TA | 0.3 | 92.4 | gel | gel | gel | gel | gel |

(continued)

| Sample Number | Post-add compound | Post-additive wt% in the sample | Initial Viscosity | Viscosity after Freeze-Thaw cycles | | | | |
|---|---|---|---|---|---|---|---|---|
| | | | | 1st cycle | 2nd cycle | 3rd cycle | 4th cycle | 5th cycle |
| 3 | TA | 0.5 | 91.4 | gel | gel | gel | gel | gel |
| 4 | TA | 1.0 | 90.3 | 99.6 | 101.2 | 119.5 | 116.5 | 109.8 |
| 5 | TA | 2.0 | 87.3 | 88.5 | 88.5 | 99.9 | 102.3 | 100.1 |
| 6 | DMTA | 0.2 | 91.7 | gel | gel | gel | gel | gel |
| 7 | DMTA | 0.3 | 89.8 | gel | gel | gel | gel | gel |
| 8 | DMTA | 0.5 | 88.9 | 105.8 | 120.0 | 143.4 | 145.6 | 132.5 |
| 9 | DMTA | 1.0 | 87.4 | 91.3 | 93.5 | 105.6 | 107.4 | 104.5 |
| 10 | DMTA | 2.0 | 85.5 | 90.2 | 88.0 | 100.3 | 102 | 100.5 |

In Table 6, the term "gel" indicates that a coagulum is formed in the paint sample post freeze-thaw and the paint cannot be reformed even after stirring the sample.

Table 7: Opacity data on paint samples with DMTA and TA additives that are subjected to 5 F/T cycles and when 0.2 wt% of DMTA is used as F/T additive and as a neutralizer.

| Paint Sample with the additive, wt% | Opacity, % (Reference) | Opacity, % (Sample) |
|---|---|---|
| TA, 1.0 wt% | 94.27 | 93.07 |
| TA, 2.0 wt% | 94.23 | 93.45 |
| DMTA, 0.5 wt% | 95.08 | 94.25 |
| DMTA, 1.0 wt% | 94.7 | 92.94 |
| DMTA, 2.0 wt% | 94.92 | 92.68 |

Data from tables 7 and 8, indicate that opacity and gloss of the paints are retained when DMTA and TA are used as F/T additives at quantities greater than 1 wt%. However, Table 9 indicates that the whiteness of the paint samples is not affected by the F/T cycles ($\Delta$E is less than 0.5) except for the case when a paint sample contains post-add TA at 1wt%.

Table 8: Gloss data on paint samples with DMTA and TA additives that are subjected to 5 F/T cycles and when 0.2 wt% of DMTA is used as neutralizer.

| Paint Sample with the additive, wt% | Gloss 20°, 65°, 85° (reference) | Gloss 20°, 65°, 85° (sample) |
|---|---|---|
| TA, 1.0 wt% | 3.4, 19.4, 31.6 | 2.6, 11.9, 14.8 |
| TA, 2.0 wt% | 3.4, 19.7, 33.2 | 4.2, 20.5, 31.9 |
| DMTA, 0.5 wt% | 3.3, 19.2, 30.7 | 2.0, 6.9, 6.8 |
| DMTA, 1.0 wt% | 3.3, 19.0, 31.9 | 2.8, 13.4, 15.0 |
| DMTA, 2.0 wt% | 3.4, 19.6, 34.3 | 4.5, 23.2, 32.6 |

Table 9: Whiteness data on paint samples with DMTA and TA additives that are subjected to 5 F/T cycles and when 0.2 wt% of DMTA is used as neutralizer.

| Paint Sample with the additive, wt% | L (ref) | L (sample) | a (ref) | a (sample) | b (ref) | b (sample) | $\Delta$E |
|---|---|---|---|---|---|---|---|
| TA, 1.0 wt% | 94.38 | 93.85 | -0.77 | -0.71 | 1.43 | 1.44 | 0.53 |

(continued)

| Paint Sample with the additive, wt% | L (ref) | L (sample) | a (ref) | a (sample) | b (ref) | b (sample) | ΔE |
|---|---|---|---|---|---|---|---|
| TA, 2.0 wt% | 94.72 | 94.51 | -0.76 | -0.74 | 1.47 | 1.48 | 0.21 |
| DMTA, 0.5 wt% | 94.38 | 94.42 | -0.76 | -0.71 | 1.38 | 1.39 | 0.06 |
| DMTA, 1.0 wt% | 94.56 | 94.48 | -0.787 | -0.71 | 1.46 | 1.46 | 0.11 |
| DMTA, 2.0 wt% | 94.53 | 94.32 | -0.76 | -0.70 | 1.60 | 1.47 | 0.25 |

Example 3

This example studies the effect of DMTA and TA as F/T additives when inorganic bases are used as neutralizers. In this case, 10% KOH is used as the neutralizer. Table 10 outlines the viscosity changes observed in paint samples. These samples do not have any glycol added to them. The blank paint solidifies after one (1) cycle making it ineffectual as paint, as has been observed with other neutralizers in the present study.

Table 10: Viscosity of different paint samples containing DMTA and TA after each F/T cycle and when 0.1 wt% of 10% KOH is used as neutralizer.

| Sample Number | Post-add compound | Post-additive wt% in the sample | Initial Viscosity | Viscosity after Freeze-Thaw cycles | | | | |
|---|---|---|---|---|---|---|---|---|
| | | | | 1st cycle | 2nd cycle | 3rd cycle | 4th cycle | 5th cycle |
| 1 | TA | 0.2 | 94.8 | gel | gel | gel | gel | gel |
| 2 | TA | 0.3 | 93 | gel | gel | gel | gel | gel |
| 3 | TA | 0.5 | 92.6 | gel | gel | gel | gel | gel |
| 4 | TA | 1.0 | 90 | 94.3 | 101.6 | 118.1 | 116.6 | 111.6 |
| 5 | TA | 2.0 | 88.7 | 86.7 | 89.7 | 100.5 | 104.1 | 99.4 |
| 6 | DMTA | 0.2 | 92.9 | gel | gel | gel | gel | gel |
| 7 | DMTA | 0.3 | 91.8 | gel | gel | gel | gel | gel |
| 8 | DMTA | 0.5 | 90.6 | gel | gel | gel | gel | gel |
| 9 | DMTA | 1.0 | 88.2 | 88.3 | 96.5 | 107.6 | 109.4 | 105.7 |
| 10 | DMTA | 2.0 | 86.8 | 86.4 | 88 | 99.7 | 101.8 | 97.2 |

In Table 10, "gel" indicates that a coagulum is formed in the paint sample post freeze-thaw and the paint cannot be reformed even after stirring the sample.

Based on the data shown, the viscosity build-up when DMTA and TA is used as F/T additive under 1.0 wt% is significantly higher and in most cases the paints do not survive all the F/T cycles. However, when DMTA and TA are added as F/T additives in quantities greater than 1 wt%, the paint samples survive all the five F/T cycles. Samples that survive the F/T cycles a subjected to more performance testing. The data is summarized in Tables 11-13. Data for the samples is relative to a reference paint, which neither has a freeze thaw additive nor does it get subjected to any freeze-thaw cycle.

Table 11: Opacity data on paint samples with DMTA and TA additives that are subjected to 5 F/T cycles and when 0.1 wt% of 10% KOH is used as neutralizer.

| Paint Sample with the additive, wt% | Opacity, % (Reference) | Opacity, % (Sample) |
|---|---|---|
| TA, 1.0 wt% | 95.59 | 94.1 |
| TA, 2.0 wt% | 95.23 | 94.23 |
| DMTA, 1.0 wt% | 95.53 | 93.97 |

(continued)

| Paint Sample with the additive, wt% | Opacity, % (Reference) | Opacity, % (Sample) |
|---|---|---|
| DMTA, 2.0 wt% | 94.74 | 92.34 |

Table 12 and 13 indicates that opacity and gloss of the paint samples are retained post F/T cycles when TA or DMTA is added at 2.0 wt%. The whiteness is retained (color difference ΔE < 0.5) for all paint samples that survive 5 F/T cycles as compared to the blank paint.

Table 12: Gloss data on paint samples with DMTA and TA additives that are subjected to 5 F/T cycles and when 0.1 wt% of 10% KOH is used as neutralizer.

| Paint Sample with the additive, wt% | Gloss 20°, 65°, 85° (reference) | Gloss 20°, 65°, 85° (sample) |
|---|---|---|
| TA, 1.0 wt% | 3.3, 18.8, 29.4 | 2.8, 12.5, 14.7 |
| TA, 2.0 wt% | 3.4, 20.0, 36.7 | 4.1, 20.3, 31.9 |
| DMTA, 1.0 wt% | 3.4, 19.8, 31.5 | 2.6, 12.3, 15.2 |
| DMTA, 2.0 wt% | 3.3, 19.3, 33.7 | 4.4, 23.5, 34.3 |

Table 13: Whiteness data on paint samples with DMTA and TA additives that are subjected to 5 F/T cycles and when 0.1 wt% of 10% KOH is used as neutralizer.

| Paint Sample with the additive, wt% | L (ref) | L (sample) | a (ref) | a (sample) | b (ref) | b (sample) | ΔE |
|---|---|---|---|---|---|---|---|
| TA, 1.0 wt% | 94.5 | 94.54 | -0.77 | -0.73 | 1.39 | 1.37 | 0.06 |
| TA, 2.0 wt% | 94.55 | 94.43 | -0.77 | -0.74 | 1.44 | 1.41 | 0.13 |
| DMTA, 1.00 wt% | 94.51 | 94.48 | -0.75 | -0.68 | 1.64 | 1.62 | 0.08 |
| DMTA, 2.0 wt% | 94.57 | 94.36 | -0.74 | -0.68 | 1.54 | 1.56 | 0.22 |

Based on this study, when AMP is used as a neutralizer lower quantities of DMTA and TA (less than 1 wt%) provide F/T stability without deterioration of the paint performances. However, the same performance was not seen when DMTA and KOH are used as the neutralizers. This synergy of AMP with either DMTA and TA is striking as formulators can significantly reduce the quantities of F/T additive in their paints. Slightly larger quantities (greater than 1 wt%) of DMTA and TA additives provide the same effects when inorganic base is used. As a result of these observations, AMP can have some additional synergistic effect on improvement of F/T stability of the paint formulations.

## Claims

1. A method for providing freeze-thaw stability to a formulation, the method comprising including in the formulation a freeze-thaw stabilizer, wherein the freeze-thaw stabilizer is a compound of formula I:

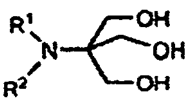

(I)

or salt thereof, wherein $R^1$ and $R^2$ are independently H, linear or branched $C_1$-$C_{10}$ alkyl, or $C_3$-$C_{12}$ cycloalkyl.

2. The method of claim 1 wherein the formulation is glycol-free.

3. The method of any one of claims 1-2 wherein the formulation is a personal care composition or is an aqueous based paint or coating that contains a binder and water.

4. The method of any one of claims 1-3 wherein the formulation further comprises a neutralizing agent selected from ammonia, 2-amino-2-methyl-1-propanol, monoethanolamine, or an inorganic base.

5. The method of any one of claims 1-4 wherein $R^1$ and $R^2$ are independently H, or linear or branched $C_1$-$C_4$ alkyl.

6. The method of any one of claims 1-5 wherein the compound of formula I is: 2-amino-2-(hydroxymethyl)propane-1,3-diol; 2-(dimethylamino)-2-(hydroxymethyl)-1,3-propanediol; 2-(diethylamino)-2-(hydroxymethyl)-1,3-propanediol; 2-(dipropylamino)-2--(hydroxymethyl)-1,3-propanediol; or 2-[bis(2-methylpropyl)amino]-2-(hydroxymethyl)-1,3-propanediol.

7. An aqueous based paint or coating comprising a freeze-thaw stabilizer, a binder, and a carrier, wherein the freeze-thaw stabilizer is a compound of formula I:

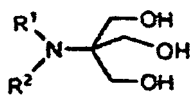

(I)

or salt thereof, wherein $R^1$ and $R^2$ are independently H, linear or branched $C_1$-$C_{10}$ alkyl, or $C_3$-$C_{12}$ cycloalkyl, and wherein the aqueous based paint or coating is glycol-free.

8. The aqueous based paint or coating of claim 7 wherein $R^1$ and $R^2$ are independently H, or linear or branched $C_1$-$C_4$ alkyl.

9. The aqueous based paint or coating of any one of claims 7-10 wherein the compound of formula I is: 2-amino-2-(hydroxymethyl)propane-1,3-diol; 2-(dimethylamino)-2-(hydroxymethyl)-1,3-propanediol; 2-(diethylamino)-2-(hydroxymethyl)-1,3-propanediol; 2-(dipropylamino)-2-(hydroxymethyl)-1,3-propanediol; or 2-[bis(2-methylpropyl)amino)-2-(hydroxymethyl)-1,3-propanediol.

10. The aqueous based paint or coating of any one of claims 7-10 further comprising a neutralizing agent selected from ammonia, amino-2-methyl-1-propanol, monoethanolamine, or an inorganic base.

11. The aqueous based paint or coating of claim 7 further comprising a neutralizing agent;
wherein:

the neutralizing agent is 2-amino-2-methyl-1-propanol; and
the freeze thaw stabilizer is 2-amino-2-(hydroxymethyl)propane-1,3-diol or 2-(dimethylamino)-2-(hydroxymethyl)-1,3-propanediol.

12. The aqueous based paint of coating of claim 11 wherein the weight ratio of 2-amino-2-(hydroxymethyl)propane-1,3-diol or 2-(dimethylamino)-2-(hydroxymethyl)-1,3-propanediol to 2-amino-2-methyl-1-propanol is from 1:1 to 5:1.

13. The aqueous based paint or coating of any one of claims 7-12 comprising 5 wt.% to 35 wt.% of the binder; 10 wt.% to 30 wt.% of a pigment; 0.2 wt.% to 10 wt.% of the compound of formula I; and 30 wt.% to 60 wt.% of the carrier.

14. The aqueous based paint or coating of any one of claims 7-13 wherein the pH is 7 to 13.

15. The aqueous based paint or coating of any one of claims 7-14 having a zero or low VOC.

## Patentansprüche

1. Verfahren zur Bereitstellung einer Frost-Tau-Stabilität für eine Formulierung, wobei das Verfahren einen in der Formulierung enthaltenen Frost-Tau-Stabilisator umfasst, wobei der Frost-Tau-Stabilisator eine Verbindung der

Formel I:

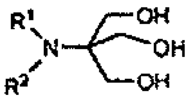

(I)

oder ein Salz davon ist, wobei $R^1$ und $R^2$ unabhängig H, lineares oder verzweigtes $C_1$-$C_{10}$-Alkyl oder $C_3$-$C_{12}$-Cycloalkyl sind.

2. Verfahren nach Anspruch 1, wobei die Formulierung glykolfrei ist.

3. Verfahren nach einem der Ansprüche 1-2, wobei die Formulierung eine Körperpflegekomposition oder eine Farbe oder Beschichtung auf Wasserbasis ist, die ein Bindemittel und Wasser enthält.

4. Verfahren nach einem der Ansprüche 1-3, wobei die Formulierung weiterhin ein Neutralisationsmittel umfasst, das aus Ammoniak, 2-Amino-2-methyl-1-propanol, Monoethanolamin oder einer anorganischen Base ausgewählt wird.

5. Verfahren nach einem der Ansprüche 1-4, wobei $R^1$ und $R^2$ unabhängig H oder lineares oder verzweigtes $C_1$-$C_4$ -Alkyl sind.

6. Verfahren nach einem der Ansprüche 1-5, wobei die Verbindung der Formel I Folgendes ist: 2-Amino-2-(hydroxymethyl)propan-1,3-diol; 2-(Dimethylamino)-2-(hydroxymethyl)-1,3-propandiol; 2-(Diethylamino)-2-(hydroxymethyl)-1,3-propandiol; 2-(Dipropylamino)-2-(hydroxymethyl)-1,3-propandiol; oder 2-[bis(2-Methylpropyl)amino]-2-(hydroxymethyl)-1,3-propandiol.

7. Farbe oder Beschichtung auf Wasserbasis, die einen Frost-Tau-Stabilisator, ein Bindemittel und einen Träger umfasst, wobei der Frost-Tau-Stabilisator eine Verbindung der Formel I:

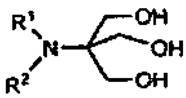

(I)

oder ein Salz davon ist, wobei $R^1$ und $R^2$ unabhängig H, lineares oder verzweigtes $C_1$-$C_{10}$-Alkyl oder $C_3$-$C_{12}$-Cycloalkyl sind und wobei die Farbe oder Beschichtung auf Wasserbasis glykolfrei ist.

8. Farbe oder Beschichtung auf Wasserbasis nach Anspruch 7, wobei $R^1$ und $R^2$ unabhängig H oder lineares oder verzweigtes $C_1$-$C_4$ -Alkyl sind.

9. Farbe oder Beschichtung auf Wasserbasis nach einem der Ansprüche 7-10, wobei die Verbindung der Formel I Folgendes ist: 2-Amino-2-(hydroxymethyl)propan-1,3-diol; 2-(Dimethylamino)-2-(hydroxymethyl)-1,3-propandiol; 2-(Diethylamino)-2-(hydroxymethyl)-1,3-propandiol; 2-(Dipropylamino)-2-(hydroxymethyl)-1,3-propandiol; oder 2-[bis(2-Methylpropyl)amino]-2-(hydroxymethyl)-1,3-propandiol.

10. Farbe oder Beschichtung auf Wasserbasis nach einem der Ansprüche 7-10, die weiterhin ein Neutralisationsmittel umfasst, das aus Ammoniak, Amino-2-methyl-1-propanol, Monoethanolamin oder einer anorganischen Base ausgewählt wird.

11. Farbe oder Beschichtung auf Wasserbasis nach Anspruch 7, die weiterhin ein Neutralisationsmittel umfasst; wobei Folgendes gilt:

das Neutralisationsmittel ist 2-Amino-2-methyl-1-propanol; und
der Frost-Tau-Stabilisator ist 2-Amino-2-(hydroxymethyl)propan-1,3-diol oder 2-(Dimethylamino)-2-(hydroxymethyl)-1,3-propandiol.

**12.** Farbe oder Beschichtung auf Wasserbasis nach Anspruch 11, wobei das Gewichtsverhältnis von 2-Amino-2-(hydroxymethyl)propan-1,3-diol oder 2-(Dimethylamino)-2-(hydroxymethyl)-1,3-propandiol zu 2-Amino-2-methyl-1-propanol zwischen 1:1 und 5:1 liegt.

**13.** Farbe oder Beschichtung auf Wasserbasis nach einem der Ansprüche 7-12, die 5 Gew.-% bis 35 Gew.-% des Bindemittels; 10 Gew.-% bis 30 Gew.-% eines Pigments; 0,2 Gew.-% bis 10 Gew.-% der Verbindung von Formel I und 30 Gew.-% bis 60 Gew.-% des Trägers umfasst.

**14.** Farbe oder Beschichtung auf Wasserbasis nach einem der Ansprüche 7-13, wobei der pH-Wert zwischen 7 und 13 liegt.

**15.** Farbe oder Beschichtung auf Wasserbasis nach einem der Ansprüche 7-14 mit einer nicht vorhandenen oder geringen VOC-Konzentration.

**Revendications**

**1.** Procédé fournissant une stabilité de congélation-décongélation à une formulation, le procédé comprenant l'incorporation dans la formulation d'un stabilisateur de congélation-décongélation, le stabilisateur de congélation-décongélation étant un composé de formule I :

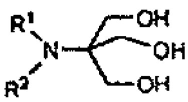

(I)

ou un sel de celui-ci, dans lequel $R^1$ et $R^2$ sont indépendamment H, un alkyle en $C_1$ à $C_{10}$ linéaire ou ramifié, ou un cycloalkyle en $C_3$ à $C_{12}$.

**2.** Procédé selon la revendication 1 dans lequel la formulation est exempte de glycol.

**3.** Procédé selon l'une quelconque des revendications 1-2 dans lequel la formulation est une composition de soin à la personne ou est une peinture ou un revêtement à base aqueuse qui contient un liant et de l'eau.

**4.** Procédé selon l'une quelconque des revendications 1 à 3 dans lequel la formulation comprend en outre un agent neutralisant choisi parmi ammoniac, 2-amino-2-méthyl-1-propanol, monoéthanolamine, ou une base inorganique.

**5.** Procédé selon l'une quelconque des revendications 1 à 4 dans lequel $R^1$ et $R^2$ sont indépendamment H, ou un alkyle en $C_1$ à $C_4$ linéaire ou ramifié.

**6.** Procédé selon l'une quelconque des revendications 1 à 5 dans lequel le composé de formule I est : 2-amino-2-(hydroxyméthyl)propane-1,3-diol ; 2-(diméthylamino)-2-(hydroxyméthyl)-1,3-propanediol ; 2-(diéthylamino)-2-(hydroxyméthyl)-1,3-propanediol ; 2-(dipropylamino)-2-(hydroxyméthyl)-1,3-propanediol ; ou 2-[bis(2-méthylpropyl)amino]-2-(hydroxyméthyl)-1,3-propanediol.

**7.** Peinture ou revêtement à base aqueuse comprenant un stabilisateur de congélation-décongélation, un liant, et un solvant le stabilisateur de congélation-décongélation étant un composé de formule I :

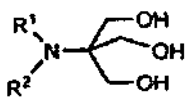

(I)

ou un sel de celui-ci, dans lequel $R^1$ et $R^2$ sont indépendamment H, un alkyle en $C_1$ à $C_{10}$ linéaire ou ramifié, ou un cycloalkyle en $C_3$ à $C_{12}$, et dans lequel la peinture ou le revêtement à base aqueuse est exempt de glycol.

**8.** Peinture ou revêtement à base aqueuse de la revendication 7 dans lequel $R^1$ et $R^2$ sont indépendamment H, ou un alkyle en $C_1$ à $C_4$ linéaire ou ramifié.

**9.** Peinture ou revêtement à base aqueuse de l'une quelconque des revendications 7 à 10 dans lequel le composé de formule I est : 2-amino-2-(hydroxyméthyl)propane-1,3-diol ; 2-(diméthylamino)-2-(hydroxyméthyl)-1,3-propanediol ; 2-(diéthylamino)-2-(hydroxyméthyl)-1,3-propanediol ; 2-(dipropylamino)-2-(hydroxyméthyl)-1,3-propanediol ; ou 2-[bis(2-méthylpropyl)amino]-2-(hydroxyméthyl)-1,3-propanediol.

**10.** Peinture ou revêtement à base aqueuse de l'une quelconque des revendications 7 à 10 comprenant en outre un agent neutralisant choisi parmi ammoniac, amino-2-méthyl-1-propanol, monoéthanolamine, ou une base inorganique.

**11.** Peinture ou revêtement à base aqueuse de la revendication 7 comprenant en outre un agent neutralisant ; dans lesquels :

l'agent neutralisant est le 2-amino-2-méthyl-1-propanol ; et
le stabilisateur de congélation-décongélation est le 2-amino-2-(hydroxyméthyl) propane-1,3-diol ou le 2-(diméthylamino)-2-(hydroxyméthyl)-1,3 -propanediol.

**12.** Peinture ou revêtement à base aqueuse de la revendication 11 dans lesquels le rapport pondéral du 2-amino-2-(hydroxyméthyl)propane-1,3-diol ou du 2-(diméthylamino)-2-(hydroxyméthyl)-1,3-propanediol au 2-amino-2-méthyl-1-propanol est de 1:1 à 5:1.

**13.** Peinture ou revêtement à base aqueuse selon l'une quelconque des revendications 7 à 12 comprenant 5 % en poids à 35 % en poids du liant ; 10 % en poids à 30 % en poids d'un pigment ; 0,2 % en poids à 10 % en poids du composé de formule I ; et 30 % en poids à 60 % en poids du solvant.

**14.** Peinture ou revêtement à base aqueuse selon l'une quelconque des revendications 7 à 13 dans lesquels le pH est de 7 à 13.

**15.** Peinture ou revêtement à base aqueuse selon l'une quelconque des revendications 7 à 14 ayant des COV égaux à zéro ou faibles.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- IN 5006CHE2012
- US 5105013 A
- US 8293002 B2